(19) Europäisches Patentamt / European Patent Office / Office européen des brevets

(11) **EP 2 413 821 B1**

(12) **EUROPEAN PATENT SPECIFICATION**

(45) Date of publication and mention
of the grant of the patent:
**10.01.2018 Bulletin 2018/02**

(51) Int Cl.:
*A61B 17/34* (2006.01)  *A61B 34/10* (2016.01)
*A61M 25/01* (2006.01)  *A61B 17/00* (2006.01)

(21) Application number: **10712156.8**

(22) Date of filing: **03.03.2010**

(86) International application number:
**PCT/IB2010/050926**

(87) International publication number:
**WO 2010/113053 (07.10.2010 Gazette 2010/40)**

(54) **HELICAL CONTINUOUS CURVATURE TUBES FOR NESTED CANNULAS**

KONTINUIERLICH GEKRÜMMTE, HELIXFÖRMIGE ROHRE FÜR TELESKOPARTIGE KANÜLE

TUBES COURBÉS D'UNE FACON CONTINUE EN FORME D'HÉLICE POUR CANULE TÉLÉSCOPIQUE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **31.03.2009  US 164945 P**

(43) Date of publication of application:
**08.02.2012  Bulletin 2012/06**

(73) Proprietor: **Koninklijke Philips N.V.**
**5656 AE Eindhoven (NL)**

(72) Inventors:
• **Elliot Eliyahu, Greenblatt**
**Briarcliff Manor**
**New York 10510-8001 (US)**

• **Aleksandra, Popovic**
**Briarcliff Manor**
**New York 10510-8001 (US)**
• **Karen Irene, Trovato**
**Briarcliff Manor**
**New York 10510-8001 (US)**

(74) Representative: **van Velzen, Maaike Mathilde**
**Philips Intellectual Property & Standards**
**High Tech Campus 5**
**5656 AE Eindhoven (NL)**

(56) References cited:
**WO-A1-2008/032230    WO-A1-2009/115936**
**WO-A2-2007/059233    WO-A2-2008/028078**
**US-A- 5 788 713**

**Description**

The present invention generally relates to nested cannula configurations that are customized for a patient to facilitate minimally invasive surgical procedures. The present invention specifically relates to an adaption of a configuration planner to employ a neighborhood of motion from a variety of arcs including helical arcs, and a construction of a nested cannula configuration including one or more helically shaped tubes and/or one or more traditionally shaped tubes (e.g., straight, circular and/or a combination thereof).

Existing navigation devices, such as catheters and bronchoscopes and other endoscopes, have several disadvantages. A particular problem encountered in bronchoscope applications is that the bronchoscope typically has a relatively large tube diameter and can only turn or be otherwise navigated at the tip. The large size is partly due to the control mechanism built within the bronchoscope that enables it to turn. As a result of their size and lack of dexterity, conventional bronchoscopes are limited in their ability to reach certain regions. For example, a typical bronchoscope can only reach the center third of a lung, where the largest airways are located. This leaves two-thirds of all lung cancers (for example) unreachable with conventional bronchoscope technology and, therefore, untreatable without major physical intervention. Even a lung biopsy, which might distinguish a benign from malignant nodule, has over a 10% chance of causing lung collapse. Thus, potentially treatable diseases are often left untreated until the disease is so aggressive that surgery is warranted and/or required.

Catheters and guidewires associated with traditional surgical techniques are relatively flexible and can reach deep within the body by following vessels. However, these devices have a tip shape designed to address the most difficult of the likely turns within the anatomy. The device's ability to maneuver through only one type of challenging turn limits the applicability of the device. Often, catheters and guidewires are often used in an 'upstream' direction, where the vessel branching requires no specific control, saving the one difficult turn for a specific location. For example, insertion of a catheter into a distal artery, such as the femoral artery (used in balloon angioplasty) toward the heart means that vessels are joining in this direction, rather than dividing. While this is effective in many cases, there is no effective mechanism to traverse complex arteries as they travel with the blood as it flows away from the heart, or along veins leading away from the heart against the flow of blood. In the lung, catheters and guidewires have relatively little control at the distal end to reach specific branches of the lung, and are therefore not suited for reaching specific targets. Insertion of a medical device such as a cannula, catheter, guidewire or scope (broncho-, endo-, etc.) can generally suffer from frictional issues and can cause tissue damage throughout the path traveled to a target. This can occur as the device is inserted into a designated anatomical region, especially when trial and error techniques through challenging anatomy cause a sawing motion. In addition, movement of the tool-tip during surgical or exploratory procedures cause motion to all of the tissue throughout the path. For example during biopsy, ablation, cautery, electrophysiology, etc., moving the tip of the device causes motion throughout the path of the device. This friction may dislodge vulnerable plaques leading to stroke, for example.

Prior techniques for moving a nested cannula were primarily focused on the interaction of multiple nested tube shapes and strengths to create a characterizable motion at the distal tip. In order to use a nested cannula by the sequential deployment of nested tubes, the configuration of the tubes must be defined so that the path can be achieved. It is not sufficient to find the midline through vessels, because this information does not describe how to break down the path into extensible, common subcomponents. For example, an S shape cannot be deployed simply as a single, continuous S shape. This is because as one end emerges from the enclosing tube, it faces in the wrong direction. Rather, two C shapes must be nested so that the first rotates counter-clockwise and the second, oriented 180 degrees from the first, extends creating a clockwise C. Further, it would require custom fabrication into the shapes, such as by heating, if they were each slightly different. Further, the diameter of the tubes must match the proposed anatomy.

International Application WO 2008/032230 A1 to Karen Trovato published March 20, 2008, and entitled "Active Cannula Configuration For Minimally Invasive Surgery" describes an effective cannula configuration system incorporating a customized tool that is created for a specific patient based on a pre-acquired 3D image, and identification of a target location. Specifically the system includes a plurality of concentric telescoping tubes nested within each other. The nested tubes are configured and dimensioned to reach a target location by generating a tube pathway through a set of arcs resulting from a three dimensional image of a particular anatomical region. The requisite image is generally obtained using a three dimensional imaging system, wherein each tubes are configured and dimensioned to reach relatively small and/or complex target locations within a particular anatomical region. The tubes may be advantageously fabricated from a material exhibiting desirable levels of flexibility/elasticity. Thus, one or more of the nested tubes may be fabricated from a Nitinol material. The Nitinol material has 'perfect memory', in that it can be bent when a force is applied, yet returns to the originally set shape once the force is removed. Nitinol can also be used within an MRI machine. It is a relatively strong material and therefore can be made thin walled, enabling the nesting of several tubes. Tubes with an outer diameter from about 5mm down to around 0.2mm are readily available in the market.

Furthermore, the three dimensional imaging system can be a CT , Ultrasound, PET, SPECT or MRI, but may also be constructed from range sensors, stereo images, video or other non-medical imaging systems. Typically, the image of the particular anatomical region is used to configure and dimension each of the plurality of tubes to define a particular shape and extension length for each of the plurality of tubes. The defined shape and extension length of each of the plurality of tubes determines whether a target location is reachable. The plurality of tubes may be configured and dimensioned to pre-set shapes and extension lengths for a particular anatomical region. The pre-set plurality of tubes can include alternating curved and straight tubes.

More particularly, the plurality of tubes are configured and dimensioned to pre-set shapes and extension lengths for a particular anatomical region associated with a particular individual. The tubes are configured and dimensioned to reach relatively small diameter locations and/or locations requiring complex maneuvers within the anatomical region. The anatomical region can be any desired region necessitating instrumental intrusion or procedure, including but not limited to thoracic regions, abdominal regions, neurological regions, cardiac regions, vascular regions, etc.

The tubes are adapted to prevent tissue damage resulting from insertion friction by creating and/or providing a barrier with an outer tube of the plurality of tubes for those tubes nested inside. The tubes can further include a medical device member or other active structure at the tip of the furthest extending tube adapted to perform and/or facilitate a medical procedure at a target location. Medical devices associated with the present invention include, but are not limited to, catheters, telescopic tips, guide wires, fiber optic devices, biopsy, suture and curatage devices, and sensors (pH, temperature, electrical). Electrical sensors are more commonly used to examine cardiac electrical function for example. The tubes can be adapted to allow manual guidance and control over the insertion of the tubes into the anatomical region aided by tactile or visual feedback. Positional feedback can also be used such as electromagnetic tracking coils embedded in the tubes or within the payload carried by the tubes. This position can be displayed on a graphical display, preferably registered to an image.

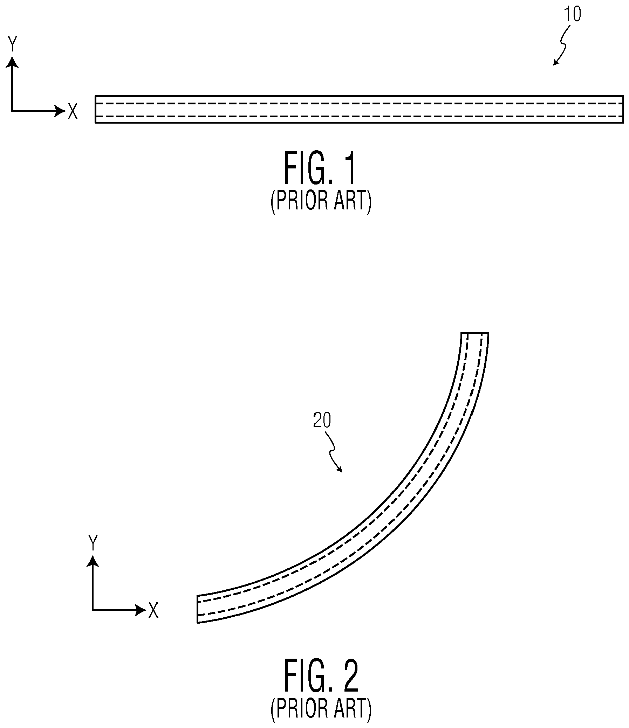

Typically, a nested cannula includes two or more tubes, preferably of a pre-designed curvature, such as for example, a straight tube 10 shown in FIG. 1 and a circular tube 20 shown in FIG. 2. The tubes are of fixed curvature in order to maintain consistent force on surrounding tubes as they are inserted, which provides a stable shape. If the tubes varied the curve or shape throughout the length, then the enclosing tube(s) would wiggle during the insertion, which is undesirable for many applications where lateral motion might cause damage or injury.

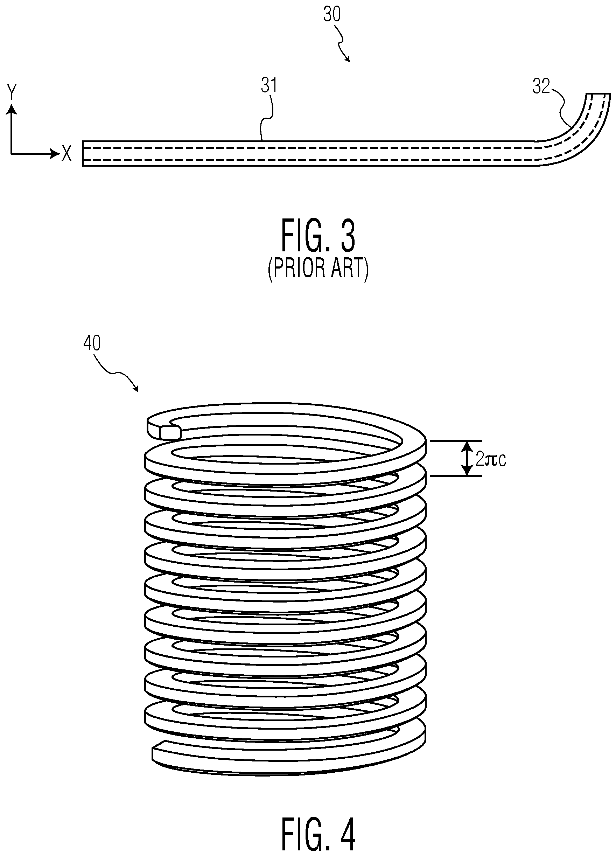

While prior devices and algorithms assume the circular tubes would be part of an arc, a manufacturing of circular tubes in a perfect circle is difficult, particularly once the length of the circular tube is greater than $2*pi*R$ (the circumference). At this length, the circular tube must be fabricated in multiple sections, circular or straight, such as for example, a tube 30 shown in FIG. 3 having a straight section 31 and a circular section 32. This creates seams between possibly inconsistent shapes, but also then requires that the tube is stored offset or in layers, similar to a wrapped garden hose, or spool of thread, particularly when all of the multiple sections are circular. As the tube is stored in this fashion, particularly for polymers, the tube can be inadvertently heated or cooled and can become miss-shaped. If the tube shape is unpredictable, then a proper set of tubes can not be pre-computed, and in addition, the nested cannula tube set will have the 'wiggle problem' as the tubes advance.

It is therefore very desirable to shape the tubes with a consistent curvature of tubes having a length greater than $2*pi*R$ (the circumference), yet ensure that they will not have to be manufactured piecewise, and will not have to be bent or wrapped into a different shape.

US Patent 5788713 discloses an apparatus with a first set of a plurality of telescoping tubes in Figures 1A-1C and a second set of a plurality of telescoping tubes in Figures 2A-2E with one or more tubes being configured and dimensioned from a single arc that may be circular or helical. In particular, this apparatus relates to calculating points within the anatomical region as basis for determining a singular or helical path adjacent all of the points.

The International Patent Application published under WO2007/059233A2 discloses a system for controlling an active cannula in Figure 1, whereby the active cannula comprises a plurality of telescoping tube with each tube being individually configured and dimensioned. Specifically, a physician defines a path in the anatomical region to reach the target location and software selects tubes from a pre-existing inventory of tubes to create a final configuration and an intermediate configuration for the active cannula.

One form of the present invention is a method for a nested cannula configuration, the method involving a reading of an image of an anatomical region; and a cooperative configuring and dimensioning of a plurality of successively smaller telescoping tubes to reach a target location relative to an anatomical region within the image through a set of arcs including one or more helical arcs, wherein each arc is determined between a point associated with the anatomical region and the target location, wherein the plurality of successively smaller telescoping tubes includes at least two helical tubes and wherein the motions performed when successively advancing each of the plurality of successively smaller telescoping tubes from a largest telescoping tube towards successively smaller telescoping tubes are designed into the plurality of successively smaller telescoping tubes so that they can perform multiple turns without the additional size or weight of motors or control wires, wherein at least two nested helical tubes form a net helix and wherein the cooperatively configuring and dimensioning the plurality of successively smaller telescoping tubes to reach the target location relative to the anatomical region within the image through the set of arcs including the at least one helical arc includes: determining a curvature of each helical tube defined by a turning radius of the helical tube and a non-zero pitch parameter of the helical tube and determining a net curvature of the net helix defined by an interaction of the curvatures of the at least two nested helical tubes, and/or determining a net torsion of the net helix defined by an interaction of the torsions of the at least two nested helical tubes.

Another form of the present invention is a nested cannula system comprising an imaging system and a configuration planner. The imaging system generates an image of an anatomical region; and the configuration planner cooperatively configures and dimensions a plurality of successively smaller telescoping tubes to reach a target location relative to an anatomical region within the image through a set of arcs including one or helical arcs, wherein each arc is determined between a point associated with the anatomical region and the target location, wherein the plurality of successively smaller telescoping tubes includes at least two helical tubes and wherein the motions performed when successively advancing each of the plurality of successively smaller telescoping tubes from a largest telescoping tube towards successively smaller telescoping tubes are designed into the plurality of successively smaller telescoping tubes so that they can perform multiple turns without the additional size or weight of motors or control wires, wherein at least two nested helical tubes form a net helix and wherein the cooperatively configuring and dimensioning the plurality of successively smaller telescoping tubes to reach the target location relative to the anatomical region within the image through the set of arcs including the at least one helical arc includes: determining a curvature of each helical tube defined by a turning radius of the helical tube and a non-zero pitch parameter of the helical tube and determining a net curvature of the net helix defined by an interaction of the curvatures of the at least two nested helical tubes, and/or determining a net torsion of the net helix defined by an interaction of the torsions of the at least two nested helical tubes.

The foregoing forms and other forms of the present invention as well as various features and advantages of the present invention will become further apparent from the following detailed description of various embodiments of the present invention read in conjunction with the accompanying drawings. The detailed description and drawings are merely illustrative of the present invention rather than limiting, the scope of the present invention being defined by the appended claims thereof.

FIG. 1. illustrates an exemplary straight tube as known in the art.
FIG. 2 illustrates an exemplary circular tube as known in the art.
FIG. 3 illustrates an exemplary straight/circular combination tube as known in the art.
FIG. 4 illustrates an exemplary embodiment of a helical tube in accordance with the present invention.
FIG. 5 illustrates an exemplary embodiment of a nested cannula system in accordance with the present invention.
FIGS. 6A and 6B illustrate an exemplary embodiment of a helical arc in accordance with the present invention.
FIG. 7 illustrates a perspective view of an exemplary three-dimensional neighborhood of arcs in accordance with the present invention.
FIG. 8 illustrates an exemplary segmentation of lung air passages and an exemplary nested cannula configuration in accordance with the present invention.
FIG. 9 illustrates an exemplary net helical tube in accordance with the present invention.
FIG. 10 illustrates a natural coordinate system determination of a helical tube in accordance with the present invention.
FIGS. 11 and 12 illustrate respective perspective and top views of an exemplary three-dimensional helical neighborhood of non-interacting helical arcs in accordance with the present invention.
FIG. 13 illustrates a perspective view of an exemplary three-dimensional helical neighborhood of interacting helical arcs in accordance with the present invention.
FIG. 14 illustrates a perspective view of an exemplary three-dimensional helical neighborhood of interacting circular arcs in accordance with the present invention.
FIG. 15 illustrates a twisting motion of a circular tube as known in the art.

The present invention provides for a nested cannula configuration system and method that generates a nested cannula customized to a patient and/or anatomical region-of-interest enabling minimally invasive surgical procedures to reach particular target locations that are commonly difficult to reach by traditional surgical means. Nitinol tubes and polymer tubes allow for flexibility and dexterity to reach complicated and challenging target locations. One or more 3D images are used to generate a series of 3D paths that define the shape and extension length of the flexible tubes. In an exemplary aspect of the present invention, tube paths are computed within a few minutes. Configured nested cannula systems and methods allow for complex vasculature to be traversed faster than manually shaped catheters that typically require trial and error to be formed correctly.

The motions required to reach a target are designed into the tool so it can perform multiple turns without the additional size or weight of motors, control wires, etc. This miniature, dexterous tool can provide accurate, minimally invasive reach into very small anatomical areas and/or regions.

According to the present invention, nested cannula systems include a plurality of telescoping, pre-shaped tubes. Concentric telescoping tubes made from flexible Nitinol (nickel- titanium alloy), or other suitable material, are generally extended along an anatomical region, each tube having a particular curvature. Nitinol is a particularly desired material for cannula fabrication due to its memory attributes and flexibility, thus enabling a tube to conform into a larger tube surrounding it until the tube is extended. Typically, the largest tube is first introduced into a desired region followed by the introduction/extension of successively smaller tubes to an expected length and orientation.

In an exemplary aspect of the present invention, tubes may be made of a polymer which is less expensive but may require thicker walls. This may be preferable if the number of tubes required is sufficiently small that they can reach the target position, or the anatomy is large enough to accommodate each tube. The characteristics of their elasticity is also important, therefore it may be advantageous to nest them near to the time that they are deployed so they have less chance to take on a new shape.

An exemplary nested cannula typically can have a plurality of telescopic Nitinol tubes (often referred to as a series of tubes) operable to reach into relatively small and/or complex locations in a desired anatomical region.

According to a beneficial aspect of the present disclosure, a nested cannula kit may include a "standard set" of tubes including one or more straight tubes of pre-designed length(s) (e.g., straight tube 10 shown in FIG. 1), one or more circular tubes of pre-designed turning radius(ii) and length(s) (e.g., circular tube 20 shown in FIG. 2), one or more straight/circular combination tubes of pre-designed turning radius(ii) and lengths (e.g., tube 20 shown in FIG. 3 having a straight portion 31 and a circular portion 32), and/or one or more helical tubes (e.g., a helical tube 40 shown in FIG. 4) of pre-designed turning radius(ii), length(s) and pitch(es). Using the "standard set" allows for reaching various locations within a given anatomical region without the cost or delay of custom manufacturing of each particular tube.

In practice, each helical tube may be manufactured under techniques for tubes and wires of uniform curvature as well known in the art. For example, one technique involves extruding a particular length of tube followed by a heat deformation of the tube around a mandrel of a particular turning radius to form a helical tube. For this example, the helix tube must have a pitch high enough for each repeated loop to clear the previous turn with the distance between adjacent loops of the helical tube. Preferably, the pitch is equal to $2\pi c$ as shown in FIG. 4 for helical tubal 40 with pitch parameter c being a non-zero constant for helical tube 40 that ensures an adequate pitch between adjacent loops of helical tube 40. In this case, the pre-designed curvature $k$ of the helical tube is in accordance with the following equation [1]:

$$k = \frac{r}{r^2 + c^2} \tag{1}$$

with $r$ being the turning radius of the mandrel and pitch parameter c being a non-zero constant that ensures an adequate pitch between adjacent loops.

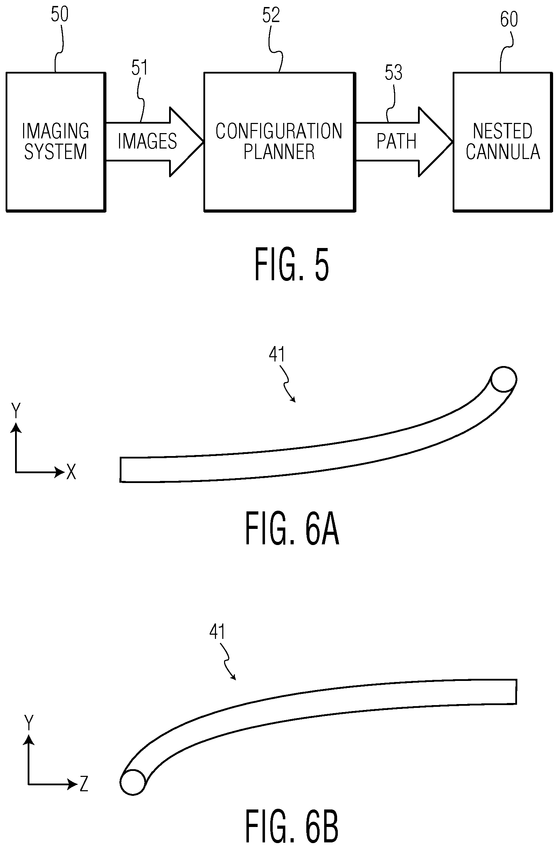

FIG. 5 illustrates an exemplary nested cannula system employing an imaging system 50 and a configuration planner 52 for configuring and dimensioning a nested cannula 60 in view of one or more helical tubes being incorporated in nested cannula 60.

Specifically, a 3D images 51 of a target anatomical region may be generated via imaging system 50 (e.g., a CT, Ultrasound, PET, SPECT, MRI, or other imaging). The images 51 may be registered to each other, creating a multi-modal image, such as, for example, PET-CT, where the PET provides critical information on the target lesions and the CT image can be segmented to define forbidden, 'critical regions', where the nested cannula may not travel. A point, typically the target, is first defined. A point can also potentially be an entry or a central key point. Starting at a point, reachable locations are calculated and a correct set of telescoping tube shapes required to reach the 3-D target locations are determined. Based on such determinations, the individual tubes are selected and/or generated.

Configuration planner 52 utilizes images 51 to cooperatively configure and dimension tubes to reach a target location relative to an anatomical region within the images 51 through a set of arcs including one or more helical arcs, such as for, example, a helical arc 41 shown in FIGS. 6A and 6B. In the following sections, key components of a framework implemented by configuration planner 52 will be described and then specified for the nested cannula application. The key components are a discretized configuration space, forbidden states, start or goal state(s), neighborhood and cost metric.

1. Configuration space:

The configuration space is defined by the span of possible parameters that describe the state, sometimes called the 'configuration' of the device. For example, a robot configuration can be defined by the angle value of each joint. The span of all possible joint angle configurations forms the configuration space. Similarly, a vehicle's configuration can be specified by its x,y position and orientation. At each state, often an array entry specified by the parameter values for one device configuration, several values are stored, including the direction to proceed from this stated to the next and the remaining cost to reach goal from this state. These values are assigned by a search method, performed later.

The configuration of a nested cannula (nested cannula) may be represented by the x,y,z location and rx,ry,rz orientation of the nested cannula's tip, resulting in a 6 dimensional problem space. Relevant locations may occur within an exemplary 12 x 12 x 29 pre-procedural CT image, with exemplary x,y,z resolutions of .078, .078 and 0.3 respectively. Discretizing all orientations at degree increments for the CT image would require 3.6 trillion states, each containing about 40 bytes, for a challenging memory requirement of 144 terabytes.

2. <u>Forbidden states:</u>

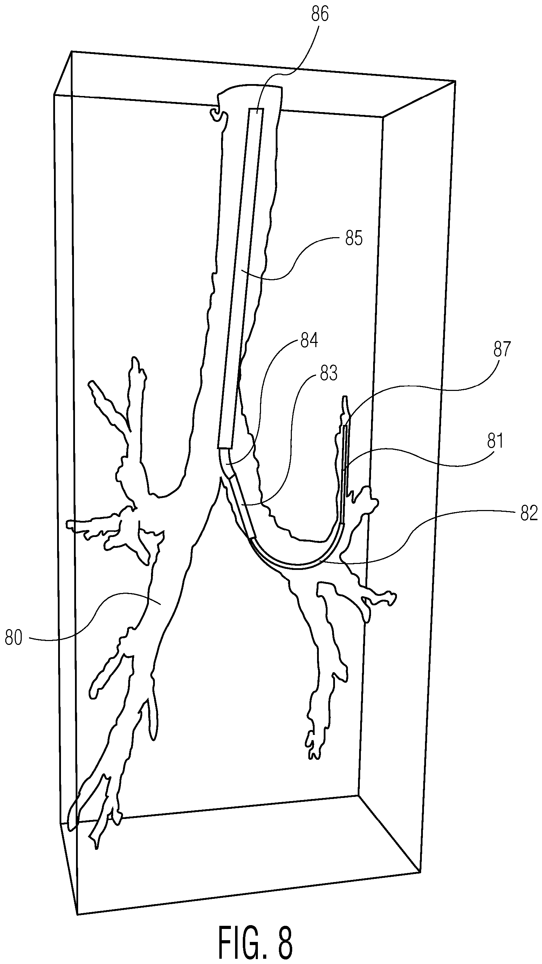

The anatomy is segmented so that some voxel regions are considered 'free- space' states and others are forbidden regions through which the device must not pass. This segmentation step can be performed by many different techniques, including manual drawing, model based segmentation where the user places a nominal model in the area of the anatomy and a computer refines the segmentation, or fully automated segmentation. In this example, configuring a nested cannula for the lung requires segmentation of the lung airways. The example image in FIG. 8 is segmented using a semi-automated Fast March (A*) method with a threshold. This generates an interior free-space volume, and an external forbidden volume (lung tissue).

3. <u>Start or goal state(s):</u>

The x,y,z location of a tumor or other target (goal) can be selected as a seedpoint for the search. Alternatively, the entry position such as a state within the trachea can be used as a seed point for the search. An orientation (rx,ry, rz) must also be defined for the seedpoint location(s).

4. <u>Neighborhood:</u>

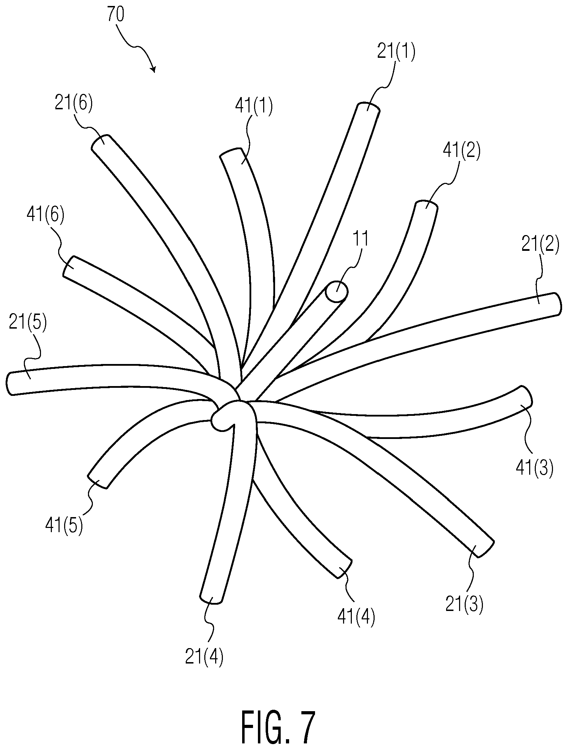

The neighborhood encapsulates the set of fundamental device motions that can be performed in free space based on the available controls and mechanical properties of a device. The curvature for a particular tube has a specified 'minimum turning radius', similar to a car. In the example neighborhood 7 shown in FIG. 7, three different curvatures are considered for the nested cannula. The first curvature is straight (no curvature, or equivalently, infinite turning radius) as embodied in straight arc 11 (corresponding to straight tube 10 shown in FIG. 1). The second curvature is circular (a finite turning radius without any pitch) as embodied by circular arcs 21 (corresponding to circular tube 20 shown in FIG. 2). The third curvature is helical (a finite turning radius with a pitch) as embodied by helical arcs 41 shown in FIG. 6. By rotating circular arc 21 and helical arc 41 in 30 degree increments, the resulting neighborhood 7 has six (6) rotations for circular arcs 21 and helical arcs 41. The length of circular arcs 21 and helical arcs 41 for a non-holonomic problem with an arbitrarily discretized space performs advantageously if the arcs are extended until the orientation is changed by 90 degrees, as shown in FIG. 7. Straight arc 11 ignores the rotational component and assumes that the incoming rotation maintains the same, since a straight tube at an arbitrary rotation follows the same path.

The neighborhood for the nested cannula is the mechanism that encapsulates the non-holonomic behavior of the device. Non-holonomic means that specific values for the control parameters (advancement plus rotation) do not uniquely define a resulting position and orientation without knowing characteristics of the path already taken. The neighborhood is a key component of a search because it captures the set of permitted motion s form a location.

In practice, circular arcs may be omitted if any portion of the search of a neighborhood would result in a length of a circular tube being greater than a circular circumference defined by a turning radius of the circular arc. For example, a circular tube may be feasible as the largest, outer diameter tube having a length less than circular circumference defined by a turning radius of the circular arc, yet impractical for any of the smaller, inner tubes. In such a case, any neighborhoods expansions during the search would omit the circular arcs.

5. <u>Cost metric:</u>

For each of the neighborhood states, a cost is assigned. This is the constituent cost for a local move based on the overall optimization criterion. In the nested cannula example, it is desired to minimize the distance traveled. Therefore, the distance traveled along the arc or straight path from a home location to a neighbor defines the cost.

Turning now to the conversion of 6D to 3D configuration space for tractability, the discretized configuration space above, requiring 144 terabytes not only causes a memory problem on most computers, but in the next section, requires a search through these states. Proceeding with this framework requires a modified technique that reduces the configuration space and computation time.

Two observations drive this modification. The first is that the forbidden region derived from the 3D CT remains the same regardless of the orientation of the tip. It is therefore useful to identify conditions under which the 3D orientation can either be ignored or reduced to a few values stored per state, within the 3D space.

The second observation results from reviewing the primary objective of the configuration space, which is to store the values describing the current state and provide directions to the next state. If an orientation can be fixed at either the start or the goal seed location, this provides an anchoring basis for calculating unique, neighboring orientations. From this seed position and orientation, positions with specific orientations can be calculated for all reachable points. Planned orientations rx,ry,rz can then be stored as values within each x,y,z configuration state along with cost and direction. Eliminating them as independent parameters of the configuration space, reduces the space from 6D to 3D, dramatically reducing the storage space required to about 77 million states and a more tractable 3 gigabytes of memory.

Positional (X,Y,Z) discretization error can also be reduced by storing the planned values within each state. The inherent (default) value of the discrete state is the value represented at the center of the voxel. Depending upon the level of discretization of the voxel, this value may be sufficient for controlling the proposed device. This may be further improved by optionally storing the precise positional (X,Y,Z) values within the state rather than incurring the discretization error throughout the configuration space. There are two specific advantages to this.

The first is that the location can be stored to arbitrary precision for the position. This can be particularly helpful when the dimensions of the voxels are not equal, which cause high precision in some directions (e.g. X and Y) with lower precision in other directions (e.g. Z). For example in a medical image such as in a CT, the voxels may be non-square or more properly, non-cubic or anisotropic, where the X and Y voxel length may be (0.078mm) and the Z voxel length (0.3mm). Although the obstacle coverage is defined with a resolution of voxels, the control can be more precisely defined by storing the computed, perhaps double precision, x,y,z,rx,ry,rz values within each state space.

The second is that if the current state is not adequately controllable to the next state, then this may be identified and automatically trigger alternate control strategies. In the simplest case, the device may stop and may wait for the proper, safe conditions to resume motion. For example, while a patient is breathing the x,y,z of the actual position of the device will move. It may be decided that only when the actual position is within 5 mm of the planned scenario, then device control may proceed.

Once these key components are defined, a shortest, collision-free path 53 is generated by configuration planner 52 from a fixed seed (start or goal) based on the set of available component tube curvatures or shapes and motions permitted with that tube (such as rotation and extension) which are encapsulated in the neighborhood. The path 53 consists of concatenated circular and/or helical arc or straight motions between the start and goal, and is carried out step-by-step with associated controls.

Concerning path generation, an A* search method may preferably be used to find all possible paths from the seed location(s). The 3D search has been described in, for example, prior applications including for vehicle maneuvering and bronchoscope maneuvering. The same 3D search may be performed for the nested cannula.

For example, FIG. 8 illustrates an example path is shown between the entry at 86 and the target 87. The path given in FIG. 8 is a schematic in order to simplify the visual results. It is noted that nested cannula must pass through the nose or mouth to reach the trachea and consider the path from the entry point 86, which has a specified orientation. The first tube is a straight tube 85 advancing a calculated length. From this point, a helical tube 84 is advanced until it reaches where helical tube 84 connects to straight tube 83. Helical tube 84 has a narrower outer diameter than the inner diameter of straight tube 85 and has a curvature specified by the neighbor and fiber selected. In a similar fashion, straight tube 83 is straight and extends until it reaches helical tube 82, which extends until it reaches straight tube 81. Each successive tube is smaller than its predecessor.

Regarding defining tube radius and helical pitch for a particular function and anatomy, a path is viable only if the series of tubes can actually fit inside a specified region. A challenge is that anatomy can be complex, varying in diameter throughout. Also, the more types of maneuvers required, the more tubes are required, and the larger diameter required at the entry. Three methods are presented to generate tube diameters based on the given path and free-space available. This is followed by a fourth, which is a preferred method of the present invention.

1. The brute force method is to create the path, and compute the required tube outer diameters for each section of tube, starting from the smallest. For each point along the path, test for illegal states between the point and a radius distance. If there is an intersection, the path is not viable, however without some additional methods this leaves the viability to luck.

2. The very safe method is to shrink the free-space by the size of the largest tube expected. In this method, every path can be realized because it is within the boundaries. Unfortunately it will also cut off access to anatomy that could be reached by small tubes.

3. The optimist's method is to shrink the free-space by the size of the smallest available tube's outer diameter. This immediately delineates the regions where no access is possible even with the smallest tube, and regions of free-space that continue to offer some potential. Planning in this space improves the chances of identifying a viable path, but still does not guarantee it.

4. An exemplary preferred method has several key steps:

4.1- Pre-compute several versions of the forbidden region. Each forbidden region is shrunk by the outside radius of each useful tube. A tube is useful only if it nests with the other tubes and the smallest is large enough to carry the intended payload or tool. The intended use of the nested cannula determines the smallest useful tube. For example, if a camera is to be inserted, it will be larger than if a fluid sample is to be taken and the tube is empty. Shrinking free-space, or equivalently, region growing the forbidden space, can be performed rapidly, and only once for each useful tube.

4.2- Choose the seed within a narrow part of the anatomy along the path. In the lung therefore, a preferred seed is likely to be a distal tumor location rather than the center of the esophagus. In the brain, the narrowest vessel should be chosen, such as an ophthalmic artery rather than the carotid artery for example. Although this is typically located at the target, it is possible to be between the target and the entry point such as in a vascular application where there is plaque buildup midway.

4.3- Set the forbidden region at the seed to be determined by the outer radius of the smallest useful tube.

4.4- Track the total number of tube changes that have occurred since the seed location. This can be stored in the configuration space in addition to the cost-to-goal. When a node is expanded, the forbidden region is selected based on the number of tube changes, which defines the radius of the current tube used. When a terminating node is reached, the radius of the required tube will also be specified.

The use of a nested cannula system according to the present invention allows clinicians and/or other medical personnel to reach/access relatively small diameter target locations and/or target locations requiring complex maneuvers within a particular anatomical region.

Nested cannula technology offers several advantages over other navigation devices including, but not limited to: (i) effective control and angulation of a telescopic tip without the use of joint motors or marionette wires; (ii) smaller tube diameter than traditional devices; (iii) cannulas that are relatively inexpensive and typically disposable; (iv) Nitinol and similar fabrication materials allow for cannulas to be formed into arbitrary shapes and curvatures, thus facilitating entry and/or access into complex regions; (v) Nitinol is an MRI friendly material; (vi) pre-formed cannula configurations can be guided manually with the assistance of image guidance and later controlled by MRI friendly piezo-motors; (vii) successively smaller concentric cannulas match various shapes for use in various medical applications which enter a larger region and ultimately reach to successively smaller regions; and (viii) early deployment of a cannula system can be achieved with manual control and accurate calculations of configurations.

In one embodiment, a standard set of cannulas can be defined such that a plurality of targets, a lung for example, can be reached using particular pattern of tubes but custom deployed at particularly calculated angles and lengths for a particular patient and/or target location. A series of helical tubes as well as straight tubes and/or circular tubes can be calculated that reach a particular target location. Target tube paths are generated from the resulting series of arcs and straight tubes. The path calculation may be weighted such that a change from one arc to another incurs an additional penalty.

In another illustrative aspect of the present invention, custom shaping ofNitinol tubes may be avoided by careful selection of a predefined set of tubes. In an exemplary system, tubes can be nested in either a set of fixed arcs, or in an alternating set of arc-straight-arc-straight tubes. Preparing appropriate predefined sets allows for simplified and speedy path calculations. Moreover, standard sets of cannulas can be produced in massive quantities rather than requiring custom shaping and manufacturing. Having a pre-set pattern enables the potential reuse of the same nested cannula system extended to different lengths to reach different target locations in the same individual in the same procedure.

Exemplary nested cannula systems and methods can be used for a variety of medical, diagnostic and/or surgical applications, including lung cancer diagnosis/biopsy and the like. For example, a nested cannula system can be used to perform a biopsy using image guidance and tracking for precision delivery of biopsy tools. A nested cannula system according to the present invention facilitates autofluorescence by using image guidance, tracking and fiber optic transmission and sensing.

Indeed, exemplary nested cannula systems and methods associated with the present invention can be utilized in lung cancer therapy for reaching target locations beyond current practice. Exemplary nested cannula systems and methods according to the present invention may also be useful in photodynamic therapy (PDT). PDT is already clinically approved and reimbursed for lung carcinoma. In an exemplary PDT procedure, an agent (e.g., Photofrin®) is injected 24-72 hours prior to therapy, accumulates at cancer sites, and is activated by light delivered within 1cm of a lesion. Unfortunately, bronchoscopes only reach the largest passages, representing about 33% of the lung. The smaller passages, where oxygen exchange occurs, cannot be reached (or reached accurately) by current techniques, systems or methods. A nested cannula system according to the present invention allows for reaching relatively smaller target locations through the use of high-resolution images and tracking In an exemplary aspect of the present invention, a nested cannula system according to the present invention may work in conjunction with current bronchoscope practice.

Exemplary nested cannula systems can be utilized for biopsy of hard to reach anatomical regions to determine the extent and/or need for molecular therapy or other intervention. It can also be utilized for 'on the spot' delivery of electronically generated radiation, e.g., using Xoft's Axxent miniaturized 2.mm X-ray source. In a cardiac environment, an exemplary nested cannula system associated with the present invention can be useful in accessing difficult locations or orientations. For vascular applications, a nested cannula system according to the present invention can reach through complex vessels currently unreachable by existing medical techniques. Moreover, the risk of dislodging clots is reduced since nested cannulas produce friction only for a portion of the entry path rather than the entire distal length.

The present invention provides for nested cannula systems that are also operable for minimally invasive surgeries for gallstones. The cannulas can be adapted to reach a gallbladder for removal. For gastroenterology, an exemplary nested cannula system according to the present invention is adapted to deliver PDT to a particular GI tract and reach target locations previously unreachable. It is also possible to reach target locations into a brain through minimally invasive vasculature.

Although this example is given in 3D, clearly the solution works for 2D images as well, with 2D neighborhoods encapsulating the device's permitted motions.

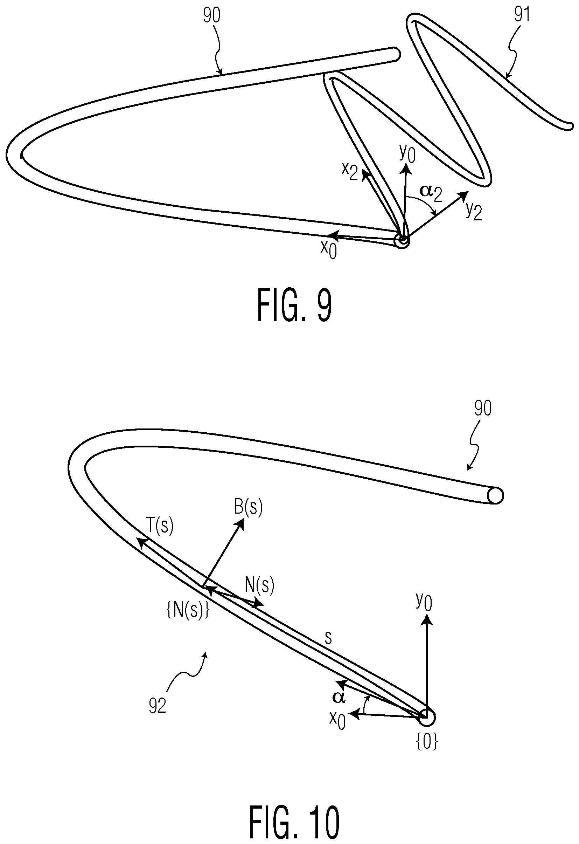

FIGS. 4-8 were described herein in a basic context for purposes of facilitating a general understanding of the configuration and dimensioning of helical tubes within a nested cannula. However, in practice, a net helix is created when multiple helical tubes are threaded together, such as, for example, a threading of a helical tube 91 within a helical tube 90 as shown in FIG. 9. The subsequent discussion herein is directed to a determination of a path of a net helix and a determination of directions of a natural coordinate system of a helical tube and net helix.

Specifically, each individual component helix is specified by its radius $r_i$ and a parameter $c_i$ related to the pitch ($c_i = Pitch/2\pi$). The subscript indicates *the $i^{th}$* of n helices. The curvature and torsion of each helix can be found from these parameters as shown in respective equations [1] and [2]:

$$\kappa_i = \frac{r_i}{r_i^2 + c_i^2} \tag{1}$$

$$\tau_i = \frac{c_i}{r_i^2 + c_i^2} \tag{2}$$

Each component helix begins at the origin of coordinate system {0} with the path starting parallel to the z-axis, and is rotated about that axis some angle $\alpha_i$, such as, for example, as shown in FIG. 10. Please note that $\alpha_i$ is zero when the oscillating plane of the $i^{th}$ helix lies in the x-y plane of {0}. The curvatures of the individual helices have both magnitude and direction. Each of the helices curvature $\kappa_i$ may be decomposed into component curvatures $\kappa_x$ and $\kappa_y$, where:

$$u_i = \begin{bmatrix} \kappa_{x,i} & \kappa_{y,i} & \tau_i \end{bmatrix}^T = \begin{bmatrix} -\kappa_i \sin(\alpha_i) & \kappa_i \cos(\alpha_i) & \tau_i \end{bmatrix}^T \tag{3}$$

The stiffness of the $i^{th}$ helix is the product of the material's Young's modulus [E], and the helix's geometrical second moment of inertia [I]. The torsional stiffness is the product the material's shear modulus [G] and the geometrical polar moment of inertia [J]. A stiffness matrix is defined by equation [4]:

$$K_i = \begin{bmatrix} E_i I_i & 0 & 0 \\ 0 & E_i I_i & 0 \\ 0 & 0 & G_i J_i \end{bmatrix} \tag{4}$$

Thus, for n tubes the net component curvatures and torsion are given by equation [5]:

$$\bar{u} = \left( \sum_{i=1}^{n} K_i \right)^{-1} \sum_{i=1}^{n} K_i u_i = \begin{bmatrix} \bar{\kappa}_x & \bar{\kappa}_y & \bar{\tau} \end{bmatrix}^T \tag{5}$$

The resulting $\bar{u}$ describes the component curvatures and torsion of the net helix. The net curvature is given by equation [6]:

$$\bar{\kappa} = \sqrt{\bar{\kappa}_x^2 + \bar{\kappa}_x^y} \tag{6}$$

The net helix can therefore be described in terms of its radius, pitch and orientation angle:

$$\overline{r} = \frac{\overline{\kappa}}{\overline{\kappa}^2 + \overline{\tau}^2} \qquad\qquad [7]$$

$$\overline{c} = \frac{\overline{\tau}}{\overline{\kappa}^2 + \overline{\tau}^2} \qquad\qquad [8]$$

$$\overline{\alpha} = \tan^{-1}\left(\frac{\overline{\kappa}_y}{\overline{\kappa}_x}\right), \qquad\qquad [9]$$

where a four quadrant inverse tangent is used

Once the properties of the net helix are found, the point on at path length s can be determined. This point, described in the {0} frame as a function of c, r, α, and s:

$$R(s)_{\{0\}} = \begin{bmatrix} R_x \\ R_y \\ R_z \end{bmatrix}_{\{0\}} \qquad\qquad [10]$$

Where:

$$R_x = -\cos(\alpha)*r*\cos(s/(r^2+c^2)^\wedge(1/2)) + \sin(\alpha)*c/(r^2+c^2)^\wedge(1/2)*r*\sin(s/(r^2+c^2)^\wedge(1/2)) - $$
$$\sin(\alpha)*(1-c^2/(r^2+c^2))^\wedge(1/2)*c*s/(r^2+c^2)^\wedge(1/2)+\cos(\alpha)*r \qquad [11]$$

$$R_y = -\sin(\alpha)*r*\cos(s/(r^2+c^2)^\wedge(1/2)) - \cos(\alpha)*c/(r^2+c^2)^\wedge(1/2)*r*\sin(s/(r^2+c^2)^\wedge(1/2)) $$
$$+\cos(\alpha)*(1-c^2/(r^2+c^2))^\wedge(1/2)*c*s/(r^2+c^2)^\wedge(1/2)+\sin(\alpha)*r \qquad [12]$$

$$R_z = (1-c^2/(r^2+c^2))^\wedge(1/2)*r*\sin(s/(r^2+c^2)^\wedge(1/2))+c^2/(r^2+c^2)*s \qquad [13]$$

A natural coordinate system {N(s)} moves along the helix and is redefined at each point along the curve, such as, for example, the natural coordinate system 92 shown in FIG. 10. The Frenet-Serret formulas determine the natural coordinate systems directions (T,N,B). The first direction (T) is the tangent to the curve, the second direction (N) points in the direction that the curve is accelerating toward, and the final direction (B) is determined by the right hand rule (B = T x N).

The three directions of {N(s)} in {0} can be given as functions of c, r, α, and s:

$$N(s)_{\{0\}} = \begin{bmatrix} N_x \\ N_y \\ N_z \end{bmatrix}_{\{0\}} \quad,\quad B(s)_{\{0\}} = \begin{bmatrix} B_x \\ B_y \\ B_z \end{bmatrix}_{\{0\}} \quad,\quad T(s)_{\{0\}} = \begin{bmatrix} T_x \\ T_y \\ T_z \end{bmatrix}_{\{0\}} \qquad [14]$$

Where:

$$N_x = \cos(\alpha)*\cos(s/(r^2+c^2)^\wedge(1/2))-\sin(\alpha)*c/(r^2+c^2)^\wedge(1/2)*\sin(s/(r^2+c^2)^\wedge(1/2)) \qquad [15]$$

$$N_y = \sin(\alpha)*\cos(s/(r^2+c^2)^\wedge(1/2)) +\cos(\alpha)*c/(r^2+c^2)^\wedge(1/2)*\sin(s/(r^2+c^2)^\wedge(1/2)) \qquad [16]$$

$$N_z=-(1-c^2/(r^2+c^2))^{(1/2)}*\sin(s/(r^2+c^2)^{(1/2)}) \qquad [17]$$

$$B_x= -\cos(\alpha)*c/(r^2+c^2)^{(1/2)}*\sin(s/(r^2+c^2)^{(1/2)}) -$$
$$\sin(\alpha)*c^2/(r^2+c^2)*\cos(s/(r^2+c^2)^{(1/2)}) -$$
$$\sin(\alpha)*(1-c^2/(r^2+c^2))^{(1/2)}*r/(r^2+c^2)^{(1/2)} \qquad [18]$$

$$B_y = -\sin(\alpha)*c/(r^2+c^2)^{(1/2)}*\sin(s/(r^2+c^2)^{(1/2)})$$
$$+\cos(\alpha)*c^2/(r^2+c^2)*\cos(s/(r^2+c^2)^{(1/2)})$$
$$+\cos(\alpha)*(1-c^2/(r^2+c^2))^{(1/2)}*r/(r^2+c^2)^{(1/2)} \qquad [19]$$

$$B_z=-(1-c^2/(r^2+c^2))^{(1/2)}*c/(r^2+c^2)^{(1/2)}*\cos(s/(r^2+c^2)^{(1/2)}) +c/(r^2+c^2)*r \qquad [20]$$

$$T_x= \cos(\alpha)*r*\sin(s/(r^2+c^2)^{(1/2)})/(r^2+c^2)^{(1/2)}$$
$$+\sin(\alpha)*c/(r^2+c^2)*r*\cos(s/(r^2+c^2)^{(1/2)} -\sin(\alpha)*(1-$$
$$c^2/(r^2+c^2))^{(1/2)}*c/(r^2+c^2)^{(1/2)} \qquad [21]$$

$$T_y = \sin(\alpha)*r*\sin(s/(r^2+c^2)^{(1/2)})/(r^2+c^2)^{(1/2)} -$$
$$\cos(\alpha)*c/(r^2+c^2)*r*\cos(s/(r^2+c^2)^{(1/2)})$$
$$+\cos(\alpha)*(1-c^2/(r^2+c^2))^{(1/2)}*c/(r^2+c^2)^{(1/2)} \qquad [22]$$

$$T_z= (1-c^2/(r^2+c^2))^{(1/2)}*r*\cos(s/(r^2+c^2)^{(1/2)})/(r^2+c^2)^{(1/2)}+c^2/(r^2+c^2) \qquad [23]$$

The homogeneous transformation between a vector in {N(s)} and the same vector described in {0}:

$$T_{\{0\}}^{\{N(s)\}} = \begin{bmatrix} N(s)_{\{0\}} & B(s)_{\{0\}} & T(s)_{\{0\}} & R(s)_{\{0\}} \\ 0 & 0 & 0 & 1 \end{bmatrix} \qquad [24]$$

The following is an exemplary code listing for implementing the aforementioned equations.

```
START CODE
clear all, close all, clc
% This program models the interaction of two helical tubes threaded
% together and plots their net shape as the outer helix is rotated. The
% helices wall thicknesses, sizes, pitches, radii and a range of
% insertion angles are specified. The program finds the analytical
% homogenous transformation as a function of these parameters and the path
% length, and then evaluates part of this transformation to plot the
% resulting paths.


%%%%%%%%%%%%%%%%%%%%%%%%%%%%%%%%%%%%%%%%%%%%%%%%%%%%%
% Controllable Parameters
%  Plot Parameters
n = 100; % set the number of points to plot for each helix
line_width = 4; % set line width
n_revolutions = .3;% set the min number of revolutions to plot


% Helices Parameters
R =  .75; %ID/OD for all tubes
Rs = .70; % IDinner/IDouter
r_vec = [10 5]; % [larger tube's helix radius, smaller tube's helix radius]
P_vec = [5 2.5]; %[LArger tube's pitch, smaller tube's pitch]
n_helixes = 6;% the number of evenly spaced helixes to plot


%%%%%%%%%%%%%%%%%%%%%%%%%%%%%%%%%%%%%%%%%%%%%%%%%%%%%
```

```
% Find the analytical homogenous transformation T_0_N between a coordinate
% system {N} at point s on a helix with c =pitch/(2*pi) and radius r at
% path length s to a coordinate system {0} fixed at the base of the helix
% if the helix is rotated some angle alpha about the z axis of {0}.
% Finally given a straight tube with a feature rotating along its path with
% some torsion tau_m that is then shaped into a helix, we find a coordinate
% system {S} in which the feature does not rotate, describes in the {0} CS.
% The analytical result is saved so that this first part of the program
% only needs to be run once; the second time around this part can be
% commented out


syms c alpha phi tau_m  real
syms r s positive


% find the frenet -seret directions in {L}
A = sqrt(c^2+r^2);
R_ = [r*cos(s/A); r*sin(s/A); c*s/A]; % define the helix in {L}
dR_ds1 = diff(R_,s);
T = simplify(dR_ds1/(dR_ds1'*dR_ds1)^(1/2))
dT_ds1 = diff(T,s);
N = simplify(dT_ds1/(dT_ds1'*dT_ds1)^(1/2))
B =simplify(cross(T,N))
signum = @(x) sign(x);


% define rotation matrices padded to be 4 by 4
Rx4 = @(theta) [ 1, 0, 0, 0; 0, cos(theta), -sin(theta), 0; 0, sin(theta), cos(theta), 0; 0, 0, 0, 1]
Ry4 = @(theta) [ cos(theta), 0, sin(theta),0 ; 0 ,1,  0, 0 ; -sin(theta), 0, cos(theta),0; 0, 0, 0, 1]
Rz4 = @(theta) [ cos(theta), -sin(theta), 0, 0 ; sin(theta), cos(theta), 0, 0; 0, 0, 1, 0;0, 0, 0, 1]
Dx4  = @(s_)[1, 0, 0, s_;  0, 1, 0, 0; 0, 0, 1, 0; 0, 0, 0, 1]


% Transform from {L} to {0}
T_0_L = Rz4(pi+alpha)*Rx4(pi/2-asin(c/A))*Dx4(-r);
% Transform from {N} to {L}
T_L_N = [[N',0]',[B',0]',[T',0]',[R_',1]']
```

```
% Transform from {N} to {0}
T_0_N = T_0_L*T_L_N


% Transform from {S) to {0}
T_0_S = T_0_N*Rz4((tau_m -c/(r^2+c^2))*s)
save('analytical_transformation.mat','T_0_N'); % write analytical matrix


%%%%%%%%%%%%%%%%%%%%%%%%%%%%%%%%%%%%%%%%%%%%%%%%%%%


% find the net helix's parameters using vectors
c_vec = P_vec/(2*pi);
k_vec = r_vec./(r_vec.^2+c_vec.^2);
tau_vec = c_vec./(r_vec.^2+c_vec.^2);
tau_bar = (tau_vec(1) + tau_vec(2)*Rs^4)/(1 +Rs^4);
alpha_vec =linspace(0,2*pi,n_helixes+1); %create a vector with all the plane angles
alpha_vec(end) =[];
kx1_vec = -k_vec(1)*sin(alpha_vec);
ky1_vec = k_vec(1)*cos(alpha_vec);
kx_bar_vec = (kx1_vec +0*Rs^4)/(1 +Rs^4);
ky_bar_vec = (ky1_vec + k_vec(2)*Rs^4)/(1 +Rs^4);
k_bar_vec = (kx_bar_vec.^2+ky_bar_vec.^2).^(1/2);
r_bar_vec = k_bar_vec./(k_bar_vec.^2 +tau_bar^2);
c_bar_vec = tau_bar./(k_bar_vec.^2 +tau_bar^2)


% add scenario where the outer tube is straight
k_str_bar = k_vec(2)*Rs^4/(1+Rs^4); % net curvature when outer tube is a straight
tau_str_bar = tau_vec(2)*Rs^4/(1+Rs^4); % net torsion when outer tube is straight


r_str_bar = k_str_bar./(k_str_bar^2 +tau_str_bar^2);
c_str_bar = tau_str_bar./(k_str_bar^2 +tau_str_bar^2);


r_bar_vec = [r_bar_vec,r_str_bar];
c_bar_vec = [c_bar_vec,c_str_bar];
```

```
alpha_bar_vec = zeros(1,n_helixes+1);
A_bar_vec = (c_bar_vec.^2+r_bar_vec.^2).^(1/2)


for i = 1:n_helixes
    alpha_bar_vec(i) = atan2(ky_bar_vec(i),kx_bar_vec(i));
end


%%%%%%%%%%%%%%%%%%%%%%%%%%%%%%%%%%%%%%%%%%%%%%%%%%%%%%%%


%Plot the net helix for each of the six angles in alpha_bar_vec


load('analytical_transformation.mat','T_0_N'); ; % load the saved analytical matrices
s_vec = linspace(0,n_revolutions*2*pi*max(A_bar_vec),n); %create a vector of lengths
p_N_0= zeros(n,3); %allocate space for points
clr = colormap(lines(n_helixes+1)); % set the colors of the helixes
for j = 1:(n_helixes+1) % for each helix
    alpha = alpha_bar_vec(j); % set the plane angle
    r= r_bar_vec(j);
    c= c_bar_vec(j);
%    A = sqrt(c^2+r^2);
    for i = 1:n % for each point
        s = s_vec(i); % set the length
        p_N_0(i,:) = eval(T_0_N(1:3,4)); % evaluate the point
    end
FIG. 9
hold on
plot3(p_N_0(:,1),p_N_0(:,2),p_N_0(:,3),'Color',clr(j,:),'LineWidth', line_width)%plot the
helix
end


%set the graph properties
FIG. 9
set(gcf,'Position',[360  388  537  534])
```

```
axis square

axis equal

set(gca,'CameraPosition',[-289.2763  219.5424  255.2470])

xlabel('x_0'),ylabel('y_0'),zlabel('z_0')

if sum(P_vec)==0

    title({'Neighborhood of 2 Interacting Rings (Pitch = 0) with';

        ['R = ',num2str(R,2),', R_s = ', num2str(Rs,2),...

        ', r_o_u_t_e_r = ', num2str(r_vec(1),3),...

        ', r_i_n_n_e_r = ', num2str(r_vec(2),3)];

        'The Outer Tube Is Rotated'});

else

    title({'Neighborhood of 2 Interacting Helices with';

        ['R = ',num2str(R,2),', R_s = ', num2str(Rs,2),...

        ', r_o_u_t_e_r = ', num2str(r_vec(1),3),...

        ', r_i_n_n_e_r = ', num2str(r_vec(2),3),...

        ', P_o_u_t_e_r = ', num2str(P_vec(1),3),...

        ', P_i_n_n_e_r = ', num2str(P_vec(2),3)];

        'The Outer Tube Is Rotated'})

end


legend('\alpha = 0^o','\alpha = 60^o','\alpha = 120^o',...

    '\alpha = 180^o', '\alpha = 240^o','\alpha = 300^o',...

    'Straight','Location', 'EastOutside')

END CODE
```

From the code above, the transformation of equation [24] is derived and stored as T_0_N.

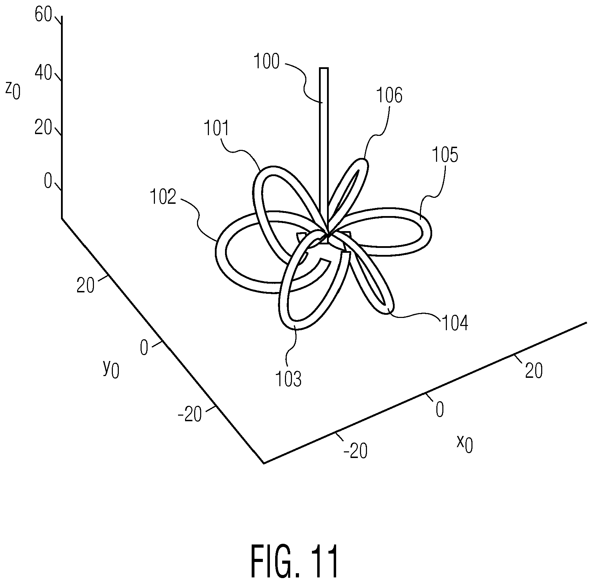

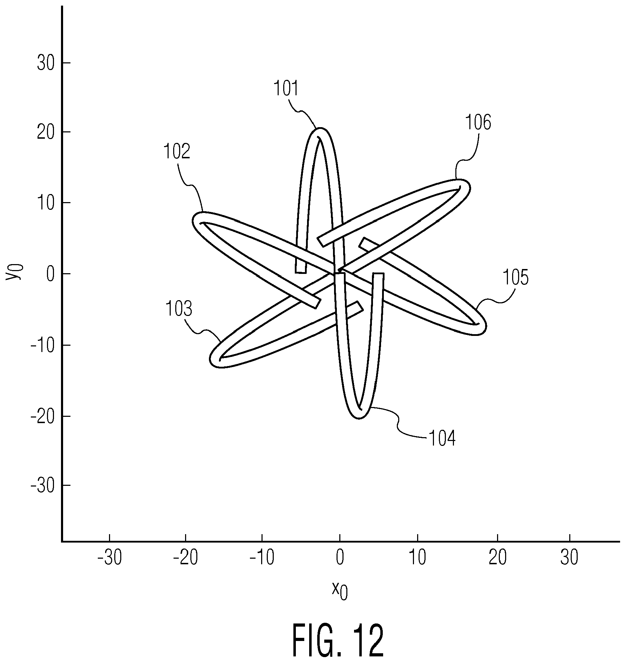

FIGS. 11 and 12 illustrates a neighborhood of six (6) helical tubes 101-106 derived from the code, where a = {0 ,60 ,120 ,180 ,240 , 300} degrees respectively in the {0} frame using R(s)$_{\{0\}}$,a s well as a straight segment 100.

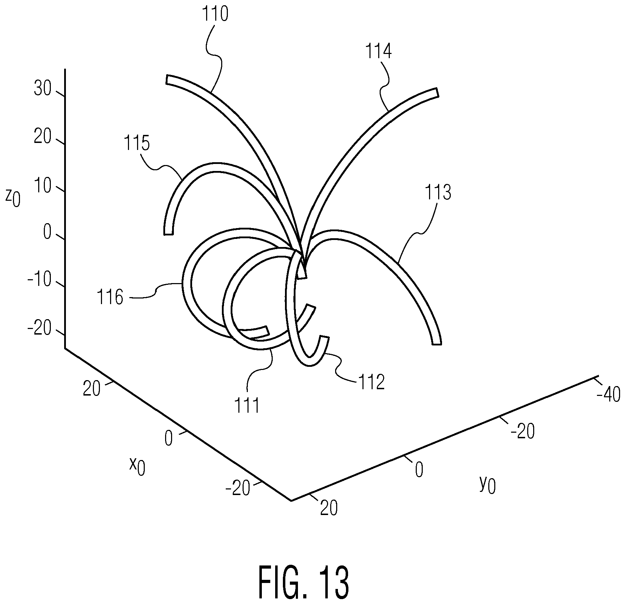

FIG. 13 illustrates a neighborhood of six (6) net helical tubes 111-116 having an interaction between an inner helical tube and an outer helical tube. The ratio of the inner diameter of the tubes to the outer diameter of the tubes is R and the ratio of a tube to the outer diameter of the tube it is inserted into is Rs. The inner tube is fixed at $\alpha$ =0, and outer tube can either be a helix at one of six different orientations ($\alpha$ = {0 ,60 ,120 ,180 ,240 , 300} degrees) respectively, or a straight segment 110. FIG. 13 highlights simulations of the code where R = 0.75 and $R_s$=0.7.

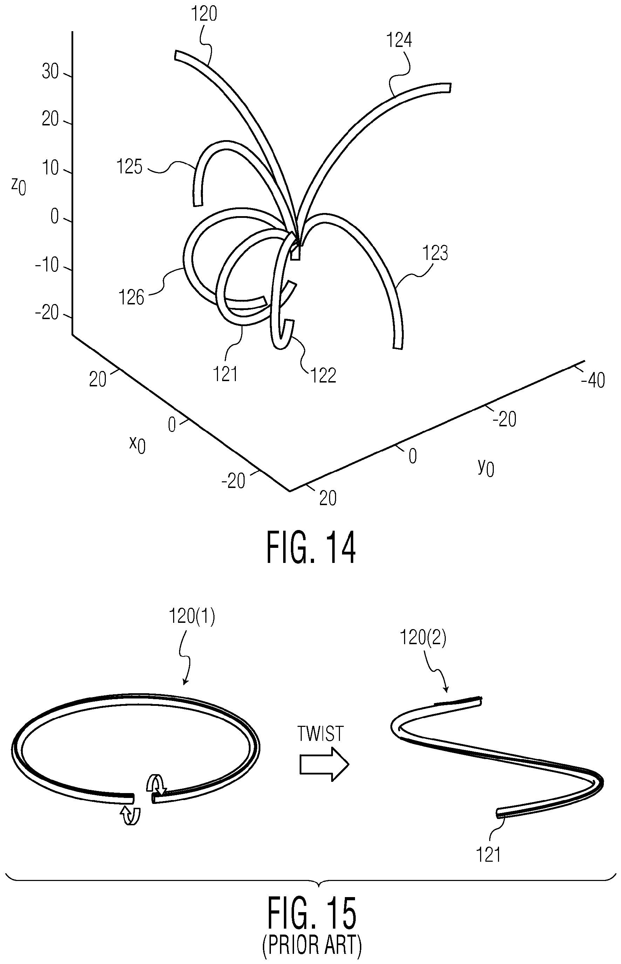

FIG. 14 illustrates a neighborhood of six (6) net circular tubes 121-126 having an interaction between two circular tubes (i.e., no pitch) where the inner tube is fixed at $\alpha$ =0 and the outer tube can either be a helix at one of six different orientations ($\alpha$ = {0 ,60 ,120 ,180 ,240 , 300} degrees) respectively or a straight segment 120. FIG. 14 highlights a simulations of the code where R = 0.75 and $R_s$ =0.7 when P_vec is set to [0 0].

In practice, helical tubes can have tools, fiducial markers, or other features whose orientation at the end of the helix is important. As previously described, every helical tube has an inherent torsion and can cause the feature to rotate along its path. This is demonstrated in FIG. 15, which demonstrates how a helical tube 120(2) may be constructed by twisting a planar ring 120(1). This twist 121 is given by the product of the torsion and the length of the curve:

$$\theta = -\overline{\tau} \cdot s = -\frac{\overline{c}}{\overline{r}^2 + \overline{c}^2}s \qquad [25]$$

This angle is the amount of rotation (in radians) that you must rotate about the T axis to correct for the twist of the helix.

The feature can be initially twisted along its path (even prior to assuming a helical shape). For a constant initial torsion (in radians per unit length) $\tau_m$, the final twist (which considers both the initial twist and the helical twist) is:

$$\theta = (\tau_m - \overline{\tau}) \cdot s = \left(\tau_m - \frac{\overline{c}}{\overline{r}^2 + \overline{c}^2}\right)s \qquad [26]$$

A coordinate system {S} can be determined that rotates along the path the amount needed to preserve the initial orientation of the feature (e.g. if the feature is at [1 0 0]$^T$ in {0} at s=o, it will always be at [1 0 0]$^T$ in {S}):

$$T_{\{0\}}^{\{S(s)\}} = T_{\{0\}}^{\{N(s)\}} \begin{bmatrix} \cos(\theta) & \sin(\theta) & 0 & 0 \\ \sin(\theta) & \cos(\theta) & 0 & 0 \\ 0 & 0 & 1 & 0 \\ 0 & 0 & 0 & 1 \end{bmatrix} = \begin{bmatrix} \hat{i}_s(s)_{\{0\}} & \hat{j}_s(s)_{\{0\}} & \hat{k}_s(s)_{\{0\}} & R(s)_{\{0\}} \\ 0 & 0 & 0 & 1 \end{bmatrix} \qquad [27]$$

Where $i_s$, $j_s$ and $k_s$ are the unit vectors in the $x_s$, $y_s$ and $z_s$ directions, respectively. This four by four homogenous transformation is generated in the above code as T_0_S.

Although the present invention has been described with reference to exemplary aspects, features and implementations, the disclosed systems and methods are not limited to such exemplary aspects, features and/or implementations. Rather, as will be readily apparent to persons skilled in the art from the description provided herein, the disclosed systems and methods are susceptible to modifications, alterations and enhancements.

## Claims

1. A method for configuring a nested cannula (60), the method comprising:

   reading an image (51) of an anatomical region; and
   cooperatively configuring and dimensioning a plurality of successively smaller telescoping tubes to reach a target location relative to an anatomical region within the image (51) through a set of arcs, wherein each arc is determined between a point associated with the anatomical region and the target location, wherein the motions performed when successively advancing each of the plurality of successively smaller telescoping tubes from a largest telescoping tube towards successively smaller telescoping tubes are designed into the plurality of successively smaller telescoping tubes so that they can perform multiple turns without the additional size or weight of motors or control wires,

   **characterised by**
   the set of arcs including at least one helical arc (41),
   wherein the plurality of successively smaller telescoping tubes includes at least two helical tubes (40), wherein at least two nested helical tubes (40) form a net helix and wherein the cooperatively configuring and dimensioning the plurality of successively smaller telescoping tubes to reach the target location relative to the anatomical region within the image (51) through the set of arcs including the at least one helical arc (41) includes:

   determining a curvature of each helical tube (40) defined by a turning radius of the helical tube (40) and a non-zero pitch parameter of the helical tube (40) and determining a net curvature of the net helix defined by an interaction of the curvatures of the at least two nested helical tubes (40), and/or
   determining a net torsion of the net helix defined by an interaction of the torsions of the at least two nested helical tubes (40).

2. The method of claim 1,

wherein the cooperatively configuring and dimensioning the plurality of successively smaller telescoping tubes to reach the target location relative to the anatomical region within the image (51) through the set of arcs including the at least one helical arc (41) further includes:

determining a torsion of each helical tube (40) defined by a turning radius of the helical tube (40) and a non-zero pitch parameter of the helical tube (40).

3. The method of claim 1,
wherein the cooperatively configuring and dimensioning the plurality of successively smaller telescoping tubes to reach the target location relative to the anatomical region within the image (51) through the set of arcs including the at least one helical arc (41) further includes:

determining a movement of a natural coordinate system along each helical tube (40).

4. The method of claim 1,
wherein the cooperatively configuring and dimensioning the plurality of successively smaller telescoping tubes to reach the target location relative to the anatomical region within the image (51) through the set of arcs including the at least one helical arc (41) further includes:

determining a torsional twist of helical tube (40).

5. A nested cannula system for configuring a nested cannula (60), comprising:

an imaging system (50) operable to generate an image (51) of an anatomical region; and
a configuration planner (52) operable to cooperatively configure and dimension a plurality of successively smaller telescoping tubes to reach a target location relative to an anatomical region within the image (51) through a set of arcs,

wherein each arc is determined between a point associated with the anatomical region and the target location, wherein the motions performed when successively advancing each of the plurality of successively smaller telescoping tubes from a largest telescoping tube towards successively smaller telescoping tubes are designed into the plurality of successively smaller telescoping tubes so that they can perform multiple turns without the additional size or weight of motors or control wires,

**characterised by**
the set of arcs including at least one helical arc (41),
wherein the plurality of successively smaller telescoping tubes includes at least two helical tubes (40), at least two nested helical tubes (40) form a net helix and wherein the cooperatively configuring and dimensioning the plurality of successively smaller telescoping tubes to reach the target location relative to the anatomical region within the image (51) through the set of arcs including the at least one helical arc (41) includes:

determining a curvature of each helical tube (40) defined by a turning radius of the helical tube (40) and a non-zero pitch parameter of the helical tube (40) and determining a net curvature of the net helix defined by an interaction of the curvatures of the at least two nested helical tubes (40), and/or
determining a net torsion of the net helix defined by an interaction of the torsions of the at least two nested helical tubes (40).

**Patentansprüche**

1. Verfahren zum Konfigurieren einer teleskopartigen Kanüle (60), wobei das Verfahren Folgendes umfasst:

Lesen eines Bilds (51) einer anatomischen Region; und
kooperatives Konfigurieren und Dimensionieren einer Vielzahl von aufeinanderfolgend kleiner werdenden Teleskoprohren zum Erreichen eines Zielorts in Bezug auf eine anatomische Region in dem Bild (51) durch eine Gruppe von Bögen,
wobei jeder Bogen zwischen einem zu der anatomischen Region gehörigen Punkt und dem Zielort bestimmt wird,
wobei die Bewegungen, die beim sukzessiven Vorschieben von jedem der Vielzahl von aufeinanderfolgend kleiner werdenden Teleskoprohren ausgehend von einem größten Teleskoprohr zu aufeinanderfolgend kleiner werdenden Teleskoprohren hin durchgeführt werden, vom Design her in die Vielzahl von aufeinanderfolgend kleiner werdenden Teleskoprohren integriert wurden, sodass sie mehrere Drehungen ohne die zusätzliche Größe oder das zusätzliche Gewicht von Motoren oder Steuerdrähten durchführen können,

**dadurch gekennzeichnet, dass** die Gruppe von Bögen mindestens einen helixförmigen Bogen (41) umfasst, wobei die Vielzahl von aufeinanderfolgend kleiner werdenden Teleskoprohren mindestens zwei helixförmige Rohre (40) umfasst,

wobei mindestens zwei verschachtelte helixförmige Rohre (40) eine Nettohelix bilden und wobei das kooperative Konfigurieren und Dimensionieren der Vielzahl von aufeinanderfolgend kleiner werdenden Teleskoprohren zum Erreichen des Zielorts in Bezug auf die anatomische Region in dem Bild (51) durch die Gruppe von Bögen einschließlich des mindestens einen helixförmigen Bogens (41) Folgendes umfasst:

Ermitteln einer Krümmung jedes helixförmigen Rohrs (40), die durch einen Drehradius des helixförmigen Rohrs (40) und einen Steigungsparameter ungleich null des helixförmigen Rohrs (40) definiert wird, und Ermitteln einer Nettokrümmung der Nettohelix, die durch eine Interaktion der Krümmungen der mindestens zwei verschachtelten helixförmigen Rohre (40) definiert wird, und/oder

Ermitteln einer Nettotorsion der Nettohelix, die durch eine Interaktion der Torsionen der mindestens zwei verschachtelten helixförmigen Rohre (40) definiert wird.

2. Verfahren nach Anspruch 1,
wobei das kooperative Konfigurieren und Dimensionieren der Vielzahl von aufeinanderfolgend kleiner werdenden Teleskoprohren zum Erreichen des Zielorts in Bezug auf die anatomische Region in dem Bild (51) durch die Gruppe von Bögen einschließlich des mindestens einen helixförmigen Bogens (41) weiterhin Folgendes umfasst:

Ermitteln einer Torsion jedes helixförmigen Rohrs (40), die durch einen Drehradius des helixförmigen Rohrs (40) und einen Steigungsparameter ungleich null des helixförmigen Rohrs (40) definiert wird.

3. Verfahren nach Anspruch 1,
wobei das kooperative Konfigurieren und Dimensionieren der Vielzahl von aufeinanderfolgend kleiner werdenden Teleskoprohren zum Erreichen des Zielorts in Bezug auf die anatomische Region in dem Bild (51) durch die Gruppe von Bögen einschließlich des mindestens einen helixförmigen Bogens (41) weiterhin Folgendes umfasst:

Ermitteln einer Bewegung eines natürlichen Koordinatensystems entlang des helixförmigen Rohrs (40).

4. Verfahren nach Anspruch 1,
wobei das kooperative Konfigurieren und Dimensionieren der Vielzahl von aufeinanderfolgend kleiner werdenden Teleskoprohren zum Erreichen des Zielorts in Bezug auf die anatomische Region in dem Bild (51) durch die Gruppe von Bögen einschließlich des mindestens einen helixförmigen Bogens (41) weiterhin Folgendes umfasst:

Ermitteln einer Torsionsverdrillung des helixförmigen Rohrs (40).

5. Verschachteltes Kanülensystem zum Konfigurieren einer verschachtelten Kanüle (60), umfassend:

ein Bildgebungssystem (50), das betriebsfähig ist, um ein Bild (51) einer anatomischen Region zu erzeugen; und einen Konfigurationsplaner (52), der betriebsfähig ist, um eine Vielzahl von aufeinanderfolgend kleiner werdenden Teleskoprohren zum Erreichen eines Zielorts in Bezug auf eine anatomische Region in dem Bild (51) durch die Gruppe von Bögen kooperativ zu konfigurieren und zu dimensionieren,
wobei jeder Bogen zwischen einem zu der anatomischen Region gehörigen Punkt und dem Zielort bestimmt wird,
wobei die Bewegungen, die beim sukzessiven Vorschieben von jedem der Vielzahl von aufeinanderfolgend kleiner werdenden Teleskoprohren ausgehend von einem größten Teleskoprohr zu aufeinanderfolgend kleiner werdenden Teleskoprohren hin durchgeführt werden, vom Design her in die Vielzahl von aufeinanderfolgend kleiner werdenden Teleskoprohren integriert wurden, sodass sie mehrere Drehungen ohne die zusätzliche Größe oder das zusätzliche Gewicht von Motoren oder Steuerdrähten durchführen können,

**dadurch gekennzeichnet, dass** die Gruppe von Bögen mindestens einen helixförmigen Bogen (41) umfasst, wobei die Vielzahl von aufeinanderfolgend kleiner werdenden Teleskoprohren mindestens zwei helixförmige Rohre (40) umfasst,

wobei mindestens zwei verschachtelte helixförmige Rohre (40) eine Nettohelix bilden und wobei das kooperative Konfigurieren und Dimensionieren der Vielzahl von aufeinanderfolgend kleiner werdenden Teleskoprohren zum Erreichen des Zielorts in Bezug auf die anatomische Region in dem Bild (51) durch die Gruppe von Bögen einschließlich des mindestens einen helixförmigen Bogens (41) Folgendes umfasst:

Ermitteln einer Krümmung jedes helixförmigen Rohrs (40), die durch einen Drehradius des helixförmigen Rohrs (40) und einen Steigungsparameter ungleich null des helixförmigen Rohrs (40) definiert wird, und Ermitteln einer Nettokrümmung der Nettohelix, die durch eine Interaktion der Krümmungen der mindestens zwei verschachtelten helixförmigen Rohre (40) definiert wird, und/oder
Ermitteln einer Nettotorsion der Nettohelix, die durch eine Interaktion der Torsionen der mindestens zwei verschachtelten helixförmigen Rohre (40) definiert wird.

**Revendications**

1. Procédé de configuration d'une canule imbriquée (60), le procédé comprenant :

la lecture d'une image (51) d'une région anatomique ; et
la configuration et le dimensionnement coopératifs d'une pluralité de tubes télescopiques successivement plus petits pour atteindre un emplacement cible par rapport à une région anatomique au sein de l'image (51) par l'intermédiaire d'un ensemble d'arcs,
dans lequel chaque arc est déterminé entre un point associé à la région anatomique et à l'emplacement cible, dans lequel
les mouvements réalisés lors de l'avancée successive de chacun de la pluralité de tubes télescopiques successivement plus petits depuis un tube télescopique le plus grand vers des tubes télescopiques successivement plus petits sont conçus dans la pluralité de tubes télescopiques successivement plus petits de sorte qu'ils puissent réaliser de multiples virages sans la taille ou le poids supplémentaire de moteurs ou de fils de commande,
**caractérisé en ce que**
l'ensemble d'arcs comporte au moins un arc hélicoïdal (41),
dans lequel la pluralité de tubes télescopiques successivement plus petits comporte au moins deux tubes hélicoïdaux (40),
dans lequel au moins deux tubes hélicoïdaux imbriqués (40) forment une hélice nette et dans lequel la configuration et le dimensionnement coopératifs de la pluralité de tubes télescopiques successivement plus petits pour atteindre l'emplacement cible par rapport à la région anatomique au sein de l'image (51) par l'intermédiaire de l'ensemble d'arcs comportant l'au moins un arc hélicoïdal (41) comportent :

la détermination d'une courbure de chaque tube hélicoïdal (40) définie par un rayon de braquage du tube hélicoïdal (40) et un paramètre de pas non nul du tube hélicoïdal (40) et la détermination d'une courbure nette de l'hélice nette définie par une interaction des courbures des au moins deux tubes hélicoïdaux imbriqués (40), et/ou
la détermination d'une torsion nette de l'hélice nette définie par une interaction des torsions des au moins deux tubes hélicoïdaux imbriqués (40).

2. Procédé selon la revendication 1,
dans lequel la configuration et le dimensionnement coopératifs de la pluralité de tubes télescopiques successivement plus petits pour atteindre l'emplacement cible par rapport à la région anatomique au sein de l'image (51) par l'intermédiaire de l'ensemble d'arcs comportant l'au moins un arc hélicoïdal (41) comportent en outre :

la détermination d'une torsion de chaque tube hélicoïdal (40) définie par un rayon de braquage du tube hélicoïdal (40) et un paramètre de pas non nul du tube hélicoïdal (40).

3. Procédé selon la revendication 1,
dans lequel la configuration et le dimensionnement coopératifs de la pluralité de tubes télescopiques successivement plus petits pour atteindre l'emplacement cible par rapport à la région anatomique au sein de l'image (51) par l'intermédiaire de l'ensemble d'arcs comportant l'au moins un arc hélicoïdal (41) comportent en outre :

la détermination d'un déplacement d'un système de coordonnées naturelles le long de chaque tube hélicoïdal (40).

4. Procédé selon la revendication 1,
dans lequel la configuration et le dimensionnement coopératifs de la pluralité de tubes télescopiques successivement plus petits pour atteindre l'emplacement cible par rapport à la région anatomique au sein de l'image (51) par l'intermédiaire de l'ensemble d'arcs comportant l'au moins un arc hélicoïdal (41) comportent en outre :

la détermination d'une vrille de torsion du tube hélicoïdal (40).

5. Système de canule imbriquée pour configurer une canule imbriquée (60), comprenant :

un système d'imagerie (50) opérationnel pour générer une image (51) d'une région anatomique ; et
un planificateur de configuration (52) opérationnel pour configurer et dimensionner de façon coopérative une pluralité de tubes télescopiques successivement plus petits pour atteindre un emplacement cible par rapport à une région anatomique au sein de l'image (51) par l'intermédiaire d'un ensemble d'arcs,
dans lequel chaque arc est déterminé entre un point associé à la région anatomique et à l'emplacement cible, dans lequel
les mouvements réalisés lors de l'avancée successive de chacun de la pluralité de tubes télescopiques successivement plus petits depuis un tube télescopique le plus grand vers des tubes télescopiques successivement plus petits sont conçus dans la pluralité de tubes télescopiques successivement plus petits de sorte qu'ils puissent réaliser de multiples virages sans la taille ou le poids supplémentaire de moteurs ou de fils de commande,
**caractérisé en ce que**
l'ensemble d'arcs comporte au moins un arc hélicoïdal (41),
dans lequel la pluralité de tubes télescopiques successivement plus petits comporte au moins deux tubes hélicoïdaux (40), au moins deux tubes hélicoïdaux imbriqués (40) forment une hélice nette et dans lequel la configuration et le dimensionnement coopératifs de la pluralité de tubes télescopiques successivement plus petits pour atteindre l'emplacement cible par rapport à la région anatomique au sein de l'image (51) par l'intermédiaire de l'ensemble d'arcs comportant l'au moins un arc hélicoïdal (41) comportent :

la détermination d'une courbure de chaque tube hélicoïdal (40) définie par un rayon de braquage du tube hélicoïdal (40) et un paramètre de pas non nul du tube hélicoïdal (40) et la détermination d'une courbure nette de l'hélice nette définie par une interaction des courbures des au moins deux tubes hélicoïdaux imbriqués (40), et/ou
la détermination d'une torsion nette de l'hélice nette définie par une interaction des torsions des au moins deux tubes hélicoïdaux imbriqués (40).

FIG. 1
(PRIOR ART)

FIG. 2
(PRIOR ART)

30

31                                    32

Y
↑
└→X

# FIG. 3
## (PRIOR ART)

40

$2\pi c$

# FIG. 4

EP 2 413 821 B1

**FIG. 5**

**FIG. 6A**

**FIG. 6B**

FIG. 7

FIG. 8

FIG. 9

FIG. 10

FIG. 11

FIG. 12

FIG. 13

FIG. 14

FIG. 15
(PRIOR ART)

**EP 2 413 821 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008032230 A1, Karen Trovato
- US 5788713 A
- WO 2007059233 A2